(19) Europäisches Patentamt
European Patent Office
Office européen des brevets

(11) Veröffentlichungsnummer: **0 018 629**
**B1**

(12) **EUROPÄISCHE PATENTSCHRIFT**

(45) Veröffentlichungstag der Patentschrift: **16.06.82**

(51) Int. Cl.[3]: **C 07 C 103/58,** C 07 C 103/64, C 07 C 102/00

(21) Anmeldenummer: **80102328.4**

(22) Anmeldetag: **30.04.80**

(54) **Verfahren zur Herstellung von N-substituierten Acrylamiden.**

(30) Priorität: **08.05.79 DE 2918486**

(43) Veröffentlichungstag der Anmeldung: **12.11.80 Patentblatt 80/23**

(45) Bekanntmachung des Hinweises auf die Patenterteilung: **16.06.82 Patentblatt 82/24**

(84) Benannte Vertragsstaaten: **AT BE CH DE FR GB IT LI LU NL SE**

(56) Entgegenhaltungen:
**GB-A- 743 302**
**US-A-2 702 822**
**US-A-3 904 635**

**C. FERRI: «Reaktionen der organischen Synthese»; Georg Thieme Verlag, Stuttgart, 1978, Seite 547**
**R. WAGNER, H. ZOOG: Synthetic Organic Chemistry (1953), Seite 568, Ref. 351**
**HOUBEN-WEYL, Band XI/2 (1958), Seiten 23–25**
**HOUBEN-WEYL, Band XI/1 (1958), Seiten 186–189**

(73) Patentinhaber: **Chemische Fabrik Stockhausen & Cie., Bäkerpfad 25, D-4150 Krefeld (DE)**

(72) Erfinder: **Bartheil, Eduard, Dipl.Chem. Dr., Minkweg 18a, D-4150 Krefeld (DE)**
Erfinder: **Dahmen, Kurt, Dipl.Chem. Dr., Von-Velsen-Strasse 6, D-4050 Mönchengladbach 2 (DE)**
Erfinder: **Goossens, Bernhard, Dipl.Chem. Dr., Am Buschkothen 53, D-5620 Velbert (DE)**
Erfinder: **Küster, Erich, Dipl.Chem. Dr., Alexanderplatz 15, D-4150 Krefeld (DE)**

(74) Vertreter: **Klöpsch, Gerald, Dr.-Ing., An Gross St. Martin 6, D-5000 Köln 1 (DE)**

EP 0 018 629 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von N-substituierten Acrylamiden

Die Erfindung betrifft ein Verfahren zur Herstellung von N-substituierten Acrylamiden.

N-substituierte Alkylacrylamide sind schon seit längerem bekannt. Sie können durch Umsetzung von Acrylnitril mit 1-Olefinen (JACS 73, 1951, 4076), sowie durch Reaktion von primären oder sekundären Aminen mit einer Additionsverbindung von Maleinsäureanhydrid und Triphenylphosphin (JS-PS 6920083) hergestellt werden. Nach GB-PS 746747 lassen sich N-substituierte Acrylamide durch Dehydrohalogenierung von β-Chlorpropionsäureamiden, nach DE-OS-23 440 70 durch Pyrolyse von β-Methoxipropionsäureamiden gewinnen. Sie können ferner nach dem Verfahren der Schotten-Baumann-Reaktion durch Umsetzung von Acrylchlorid mit entsprechenden Diaminen (US-PS-2 951 907), durch katalytische Addierung von funktionalisierten Aminen an Acetylen unter CO-Atmosphäre (US-PS-2 773 063), durch reduktive Aminierung von Diacetonacrylamid (J. Polym. Sci. 10 [1972], 595), sowie durch Pyrolyse von Norbornenderivaten (DE-OS-2 354 602) hergestellt werden. Schliesslich erhält man diese Verbindung auch nach den Verfahren der DE-OS-2 502 247, DE-OS-2 656 682 und US-PS-3 878 247, indem man an Acryl- bzw. Methacrylsäureester unter gleichzeitiger Aminolyse anlagert, wobei N-substituierte β-Aminopropionsäureamide entstehen, die pyrolytisch zu den entsprechenden α, β-ungesättigten, N-substituierten Carbonsäureamiden gespalten werden. Die GB-A-743 302 beschreibt die Herstellung von N-substituierten Carbonsäureamiden durch Umamidierung von β-Propiolakton, einem inneren Ester, und anschliessende Wasserabspaltung aus dem Hydracrylsäureamid, einem Alkohol. Die Umamidierung von Carbonsäureamiden durch Umsetzung mit substituierten Aminen unter Ammoniakeliminierung zu entsprechenden N-substituierten Carbonsäureamiden ist aus Houben-Weyl, Methoden der organischen Chemie, Band XI/2, Stuttgart 1958, Seiten 23–25 bekannt.

Sämtliche bekannten Verfahren arbeiten mit teuren bzw. hochtoxischen Ausgangsmaterialien, erfordern einen hohen Aufwand und liefern meist nur bescheidene Ausbeuten. Erwünscht wäre daher ein technisch einfaches Verfahren, das die α, β-ungesättigten N-substituierten Carbonsäureamide in hohen Ausbeuten liefert.

Gegenstand der Erfindung ist daher ein Verfahren zur Herstellung von N-substituierten Acrylamiden der allgemeinen Formel

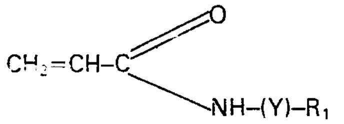

I

worin Y einen zweiwertigen gerad- oder verzweigtkettigen Alkylrest mit 2 bis 30, vorzugsweise 2 bis 18, insbesondere 2 bis 6 Kohlenstoffatomen oder einen Cycloalkylenrest mit 5 oder 6 Kohlenstoffatomen im Ring und $R_1$ Wasserstoff oder den Rest eines Amins der Formel $-N(R_2)(R_3)$, wobei $R_2$ und $R_3$ für Alkylreste mit 1 bis 4 Kohlenstoffatomen stehen, bedeuten, das dadurch gekennzeichnet ist, dass man Dihydracrylsäureamid der allgemeinen Formel

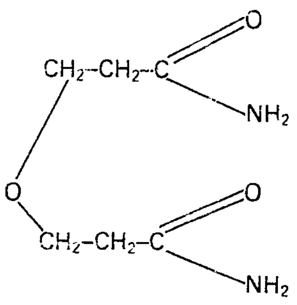

II

mit Aminen der allgemeinen Formel

$$H_2N-(Y)-R_1 \qquad III$$

in der Y und $R_1$ die oben angegebenen Bedeutungen haben unter Ammoniakeliminierung in einem Temperaturbereich von 100° bis 200° C umamidiert und die entstandenen N-substituierten β-Carbonsäureamide bei erhöhten Temperaturen unter Wasserabspaltung in die gewünschten N-substituierten Acrylamide umwandelt. Die Umwandlung der in der ersten Stufe der Reaktion entstandenen N-substituierten Säureamide in die N-substituierten Acrylamide unter Wasserabspaltung wird bevorzugt in Gegenwart eines Katalysators vorgenommen.

Wenn Y ein verzweigtkettiger Rest ist, so hat dieser bevorzugt die Formel

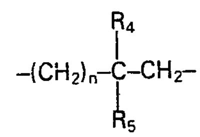

worin $R_4$ und $R_5$ niedere Alkylgruppen mit 1 bis 4 C-Atomen, vorzugsweise Methylgruppen sind und n eine Zahl von 0 bis 10 darstellt.

Der Rest kann aber auch ein Cycloalkylenrest mit 5 oder 6 C-Atomen im Ring sein.

Der Cycloalkylrest kann dabei ggf. substituiert sein, beispielsweise durch Alkyl. Wenn $R_1$ für den Rest eines Amins der Formel $N(R_2)(R_3)$ steht, so können diese Reste $R_2$ und $R_3$, die gleich oder verschieden sind, für gerad- oder verzweigtkettige Alkylenreste mit 1 bis 4 C-Atomen, beispielsweise Methyl, Ethyl, Propyl, Isopropyl oder N-Butyl stehen. Falls $R_1$ für den Rest eines Amins steht, so ist es sehr vorteilhaft, wenn das Kohlenstoffatom in β-Stellung zur Aminogruppe vollständig substituiert ist, da dann unter den Bedingungen der Etherspaltung keine β-Eliminierung stattfinden kann. Andernfalls enthält das hergestellte n-substituierte Acrylamid in geringen Mengen eine Verbindung, die anstelle der Aminogruppen eine Doppelbindung besitzt, was sich bei der Weiterverwendung der Produkte schädlich auswirkt, da

diese Verbindung z.B. bei der Polymerisation einen Vernetzer darstellt.

Das Dihydracrylsäureamid stellt den Sonderfall eines β-alkoxisubstituierten Propionamids dar. Bei der katalytischen Etherspaltung bei erhöhten Temperaturen entsteht N-substituiertes Acrylamid und Alkohol, in diesem Falle N-substituiertes Hydrocrylsaureamid, das unter den gewählten Reaktionsbedingungen weiteres N-substituiertes Acrylamid und Wasser ergibt. Im Gegensatz zu Alkoholen ist Wasser bei dieser Reaktion chemisch inert und neigt nicht zur Rückreaktion, die bei einer Alkoholabspaltung verhindert werden muss, indem man ihn – destillativ – aus dem Gemisch entfernt, wobei der Sumpf längere Zeit thermisch beansprucht wird.

Nachdem die erfindungsgemäss verwendeten Dihydracrylsäureamide trifunktionelle Verbindungen darstellen die sowohl an der Ethergruppe als auch an beiden Amidgruppen Reaktionen eingehen können und es zum Beispiel aus Houben-Weyl, Band XI/1, Seite 188–189 bekannt ist, dass Ether durch β-ständige Carboxylgruppen derart aktiviert werden, dass ein Austausch gegen Amin bereits bei 60° C bevorzugt abläuft, während die Amidgruppe erst ab ca. 100° C in Reaktion tritt, war nicht zu erwarten, dass bei Temperaturen von 100 bis 200° C praktisch kein Austausch, sondern lediglich Umamidierung stattfindet.

Das als Ausgangsverbindung für die Umamidierung eingesetzte Dihydracrylsäureamid kann leicht aus Dihydracrylsäurenitril (Bis-(2-Cyanoethyl)-ether) durch katalysierte Wasseranlagerung (z.B. nach DE-OS-20 65 667) oder Verseifung mit Natronlauge/Wasserstoffperoxid (vgl. A. P. Terentjev, A. N. Kost und A. M. Berlin, J. Obšč. Chim. 26 [1956], 827–830) erhalten werden. Die Verbindung fällt aus Wasser nach Einengen kristallin aus. Dihydriacrylsäurenitril ist sowohl durch Anlagerung von Wasser an zwei Moleküle als auch von Hydracrylsäurenitril (Ethylencyanhydrin, 3-Hydroxipropionitril) an ein Molekül Acrylnitril unter basischer Katalyse zugänglich (vgl. O. Bayer, Angew. Chem. 61 [1949], 229–241).

Die Umamidierung kann bei Normaldruck mit und ohne Zusatz von Katalysatoren durchgeführt werden. Als Katalysatoren sind besonders Säuren geeignet, von denen sich als besonders vorteilhaft Essigsäure in Mengen von etwa 0,5 bis 1,0 Mol–% gezeigt hat.

Die Umamidierung (erste Stufe) wird bevorzugt in einem Temperaturbereich von 130 bis 175° C vorgenommen.

Anschliessend an die Umamidierung wird aus dem gebildeten N,N'-disubstituierten Dihydracrylsäureamid pyrolytisch Wasser abgespalten, worauf das gebildete N-substituierte Acrylamid isoliert wird. Die Pyrolyse wird vorzugsweise in Gegenwart von sauren oder basischen Substanzen entweder diskontinuierlich oder kontinuierlich vorgenommen. Bei diskontinuierlicher Arbeitsweise erfolgt die Pyrolyse bei 70 bis 200° C, vorzugsweise bei 90° C bis 150° C in der Flüssigphase. Als saure oder basische Katalysatoren können z.B. Schwefelsäure, Phosphorsäure oder Polyphosphorsäure dienen, als basische Katalysatoren finden z.B. Natrium- oder Kaliumhydroxid bzw. -karbonat oder Kalzium- bzw. Bariumoxid Verwendung.

Bei kontinuierlicher Arbeitweise wird die Pyrolyse bei 250° C bis 450° C in der Gasphase vorgenommen. Hierbei wird das umamidierte Produkt schonend, vorteilhaft im Vakuum, z.B. mit Hilfe eines Dünnschichtverdampfers verdampft, worauf die Dämpfe durch ein auf 250° C bis 450° C erhitztes, mit einem festen Dehydratisierungskatalysator gefülltes Reaktionsrohr geleitet werden, wo die Spaltung in Wasser und N-substituiertes Acrylamid stattfindet. Als Katalysator bei dieser Verfahrensweise werden anorganische Oxide mit saurem oder basischem Charakter, wie z.B. Aluminiumoxid, Siliziumoxid oder Bariumoxid verwendet, die gegebenenfalls noch imprägniert sein können.

Dehydratisiertes Produkt und Reaktionswasser werden anschliessend aufgefangen bzw. fraktioniert kondensiert.

Die Verfahrensprodukte sind für viele Zwecke gewerblich verwertbar; Beispiele hierfür sind in der DE-OS-2 344 070 angegeben. Die Produkte können u.a. auch polymerisiert werden und sind in Form ihrer Polymerisate bzw. Copolymerisate mit anderen geeigneten Copolymeren, gegebenenfalls nach Quarternisierung als Flockungs- und Retentionshilfsmittel geeignet.

Die Erfindung wird durch die folgenden Beispiele erläutert:

Beispiel 1

N-(3-Dimethylaminopropyl) acrylamid

Man erhitzt 320,4g (2,0 Mol) Dihydracrylsäureamid mit 429,2g (4,2 Mol) 3-Dimethylaminopropylamin und 4ml Eisessig 8 Stunden über ein Temperaturintervall von 135 bis 160° C bis zum Ende der Ammoniakentwicklung. Man gibt 4,5g Orthophosphorsäure und 9,0g Polyphosphorsäure hinzu und destilliert bei $Kp_{10}$=142 bis 152° C 534g Flüssigkeit. Bei der Redestillation erhält man nach einem Vorlauf von 36g Wasser 398g (2,6 Mol=64% d.Th.) Produkt mit $Kp_{0,1}$=102 bis 104° C.

NMR ($CDCl_3$):$\delta$=1,55 bis 1,95 (m,2); 2,25 (s,6); 2,4 (t,2); 3,1 bis 3,55 (m,2); 5,45 bis 6,3 (m,3).

Beispiel 2

N-(N',N', 2,2-Tetramethyl-3-aminopropyl) acrylamid

Man erhitzt 320,4g (2,0 Mol) Dihydracrylsäureamid mit 546,8g (4 Mol) N,N,2,2-Tetramethylpropandiamin-1,3 (Dimethylaminoneopentylamin) und 4ml Eisessig 8 Stunden über ein Temperaturintervall vom 145 bis 170° C bis zum Ende der Ammoniakentwicklung. Man gibt 4,5g Orthophosphorsäure und 9,0g Polyphosphorsäure hinzu und destilliert bei $Kp_{10}$=140 bis 150° C 557g Flüssigkeit. Bei der Redestillation erhält man nach einem Vorlauf von Wasser 430g (2,3 Mol=58% d.Th.) Produkt mit $Kp_{0,1}$=94 bis 98° C.

NMR ($CDCl_3$):$\delta$=0,9 (s,6); 2,3 (m,8); 3,15 (d,2); 5,3 bis 6,5 (m,3); 8,0 (m,1).

Beispiel 3

N-(3-Dimethylamino-propyl) acrylamid

Man erhitzt 320,4g (2,0 Mol) Dihydracrylsäureamid mit 429,2g (4,2 Mol) 3-Dimethylaminopropylamin und 4ml Eisessig 8 Stunden über ein Temperaturintervall von 135 bis 160° C bis zum Ende der Ammoniakentwicklung. Anschliessend zieht man alle flüchtigen Bestandteile im Hochvakuum bis 100° C ab, wobei 660g gelbbraunes Öl resultiert, das bei Raumtemperatur kristallin erstarrt.

NMR ($CDCl_3$): $\delta$=1,45 bis 2,0 (q,4); 2,1 bis 2,6 (m,8); 2,25 (s,12); 3,1 bis 3,6 (q,4); 3,7 (t,4); 7,2 (m,2).

660g (2,0 Mol) N,N'-disubstituiertes Dihydracrylsäureamid werden aufgeschmolzen, sukzessiv im Dünnschichtverdampfer (Temperatur 300° C, Vakuum 0,2 mbar) verdampft und die Dämpfe durch ein Reaktionsrohr (100cm lang, 3cm Ø) geleitet, das mit Aluminiumoxid (Kugeln, 5–8mm) gefüllt ist und von aussen mittels einer Heizbandage auf 300° C geheizt wird. Während des zweistündigen Prozesses werden 525g (3,4 Mol=84% d.Th.) N-(3-Dimethylamino-propyl) acrylamid gewonnen.

Beispiel 4

N-(N',N',2,2-Tetramethyl-3-aminopropyl)acrylamid

Man erhitzt 320,4g (2,0 Mol) Dihydracrylsäureamid mit 546,8g (4,2 Mol) N,N,2,2-Tetramethylpropandiamin-1,3 und 4ml Eisessig 8 Stunden über ein Temperaturintervall von 145 bis 170° C bis zum Ende der Ammoniakentwicklung. Anschliessend zieht man alle flüchtigen Bestandteile im Hochvakuum bis 100° C ab, wobei 773g gelbbraunes Öl resultiert.

NMR ($CDCl_3$): $\delta$=1,1 (s,12); 2,1 bis 2,7 (m,8); 2,3 (s,12); 3,15 (t,4); 7,75 (m,2)

773 g (2,0 Mol) N,N'-disubstituiertes Dihydracrylsäureamid werden sukzessiv im Dünnschichtverdampfer (Temperatur 300° C, Vakuum 0,2 mbar) verdampft und die Dämpfe durch ein Reaktionsrohr (100cm lang, 3cm Ø) geleitet, das mit Aluminiumoxid (Kugeln, 5–8mm) gefüllt ist und von aussen mittles einer Heizbandage auf 300° C geheitzt wird. Innerhalb von etwa 2 Stunden werden 645g (3,5 Mol=88% d.Th.) N-(N',N',2,2-Tetramethyl-3-aminopropyl) acrylamid gewonnen.

**Patentansprüche**

1. Verfahren zur Herstellung von N-substituierten Acrylamiden der allgemeinen Formel

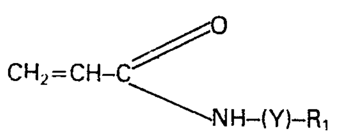

I

worin Y einen zweiwertigen gerad- oder verzweigtkettigen Alkylenrest mit 2 bis 30, vorzugsweise 2 bis 18, insbesondere 2 bis 6 Kohlenstoffatomen oder einem Cycloalkylenrest mit 5 oder 6 Kohlenstoffatomen im Ring und $R_1$ Wasserstoff oder den Rest eines Amins der Formel -N ($R_2$) ($R_3$), wobei $R_2$ und $R_3$ für Alkylreste mit 1 bis 4 Kohlenstoffatomen stehen, bedeuten, dadurch gekennzeichnet, dass man Dihydracrylsäureamid der allgemeinen Formel

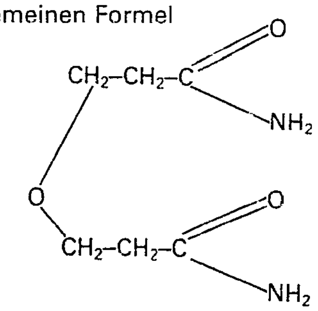

II

mit Aminen der allgemeinen Formel

$H_2N{-}(Y){-}R_1$ III

in der Y und $R_1$ die oben angegebene Bedeutung haben unter Ammoniakeliminierung in einem Temperaturbereich von 100° bis 200° C umamidiert und die entstandenen N-substituierten β-Carbonsäureamide bei erhöhten Temperaturen unter Wasserabspaltung in die gewünschten N-substituierten Acrylamide umwandelt.

2. Verfahren nach Anspruch 1, dadurch gekennzeichnet, dass man die Umamidierung in einem Temperaturbereich von 130 bis 175° C vornimmt.

3. Verfahren nach Ansprüchen 1 bis 2, dadurch gekennzeichnet, dass man die Umamidierung in Gegenwart von katalytischen Mengen Säure vornimmt.

4. Verfahren nach Anspruch 3, dadurch gekennzeichnet, dass man die Umamidierung in Gegenwart von Essigsäure vornimmt.

5. Verfahren nach Ansprüchen 1 bis 4, dadurch gekennzeichnet, dass man die Wasserabspaltung in Gegenwart von sauren oder basischen Substanzen vornimmt.

6. Verfahren nach Ansprüchen 1 bis 5, dadurch gekennzeichnet, dass man die Wasserabspaltung diskontinuierlich bei Temperaturen von 70° bis 200° C vornimmt.

7. Verfahren nach Ansprüchen 1 bis 5, dadurch gekennzeichnet, dass man die Wasserabspaltung kontinuierlich bei Temperaturen von 250° bis 450° C vornimmt.

8. Verfahren nach Anspruch 7, dadurch gekennzeichnet, dass man das umamidierte Produkt in der Gasphase dehydratisiert.

9. Verfahren nach Ansprüchen 7 und 8, dadurch gekennzeichnet, dass man das umamidierte Produkt schonend verdampf und die Dämpfe über einem festen Katalysator dehydratisiert.

10. Verfahren nach Ansprüchen 7 bis 9, dadurch gekennzeichnet, dass man als feste Katalysatoren anorganische saure oder basische Oxide, vorzugsweise $Al_2O_3$, $SiO_2$ oder BaO verwendet.

**Claims**

1. Process for the manufacture of N-substituted acrylamides of the general formula

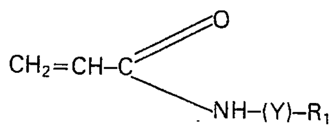

I

in which

Y represents a divalent straight-chain or branched alkylene radical having from 2 to 30, preferably from 2 to 18, and especially from 2 to 6, carbon atoms or a cycloalkylene radical having 5 or 6 carbon atoms in the ring, and

$R_1$ represents hydrogen or the radical of an amine of the formula -N ($R_2$) ($R_3$), $R_2$ and $R_3$ denoting alkyl radicals having from 1 to 4 carbon atoms, characterised in that dihydro-acrylic acid amide of the general formula

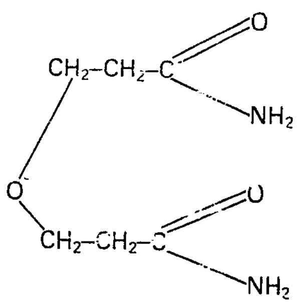

II

is transamidated with amines of the general formula

$H_2N-(Y)-R_1$ III

in which Y and $R_1$ have the meanings given above, with the elimination of ammonia in a temperature range of from 100° to 200° C, and the resulting N-substituted β-carboxylic acid amides are converted into the desired N-substituted acrylamides at elevated temperature and with the splitting off of water.

2. Process according to claim 1, characterised in that the transamidation is carried out in a temperature range of from 130 to 175° C.

3. Process according to claims 1 to 2, characterised in that the transamidation is carried out in the presence of catalytic amounts of acid.

4. Process according to claim 3, characterised in that the transamidation is carried out in the presence of acetic acid.

5. Process according to claims 1 to 4, characterised in that the splitting off of water is effected in the presence of acidic or basic substances.

6. Process according to claims 1 to 5, characterised in that the splitting off of water is effected discontinuously at temperatures of from 70° to 200° C.

7. Process according to claims 1 to 5, characterised in that the splitting off of water is effected continuously at temperatures of from 250° to 450° C.

8. Process according to claim 7, characterised in that the transamidated product is dehydrated in the gas phase.

9. Process according to claims 7 and 8, characterised in that the transamidated product is evaporated under mild conditions and the vapours are dehydrated over a solid catalyst.

10. Process according to claims 7 to 9, characterised in that as solid catalysts there are used inorganic acidic or basic oxides, preferably $Al_2O_3$, $SiO_2$ or BaO.

**Revendications**

1. Prodédé de préparation d'amides acryliques N-substitués de la formule générale

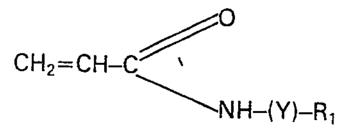

I

dans laquelle

Y désigne un groupe alcoylène à chaîne droite ou ramifiée bivalent en $C_2$ à $C_{30}$, de préférence en $C_2$ à $C_{18}$ et en particulier en $C_2$ à $C_6$, ou en groupe cyclo-alcoylène avec un noyau à 5 ou 6 atomes de carbone, et

$R_1$ représente un atome d'hydrogène ou un groupe amine de la formule -N ($R_2$) ($R_3$), dans laquelle $R_2$ et $R_3$ désignent des radicaux alcoyle en $C_1$ à $C_4$, caractérisé en ce que l'on soumet l'amide dihydracrylique de la formule

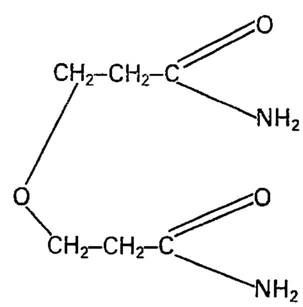

II

à une transamidation dans la gamme de températures de 100 à 200° C, avec élimination d'ammoniac, par réaction avec une amine de la formule générale

$H_2N-(Y)-R_1$ III

dans laquelle Y et $R_1$ possèdent la signification définie ci-dessus, l'amide d'acide β-carboxylique N-substitué formé étant transformé à température accrue, par élimination d'eau, en acryl-amide N-substitué recherché.

2. Procédé suivant la revendication 1, caractérisé en ce que la transamidation est réalisée dans une gamme de températures de 130 à 175° C.

3. Procédé suivant l'une des revendications 1 et 2, caractérisé en ce que la transamidation est effectuée en présence d'une quantité catalytique d'une acide.

4. Procédé suivant la revendication 3, caractérisé en ce que la transamidation est effectuée en présence d'acide acétique.

5. Procédé suivant l'une des revendications 1 à 4, caractérisé en ce que l'élimination d'eau est réalisée en présence de substances acides ou basiques.

6. Procédé suivant l'une des revendications 1 à 5, caractérisé en ce que l'élimination d'eau est realisée de manière discontinue entre 70 et 200° C.

7. Procédé suivant l'une des revendications 1 à 5, caractérisé en ce que l'élimination d'eau est réalisée de manière continue entre 250 et 450° C.

8. Procédé suivant la revendication 7, caractérisé en ce que le composé transamidé est déshydraté en phase gazeuse.

9. Procédé suivant l'une des revendications 7 et 8, caractérisé en ce que le composé transamidé est vaporisé dans des conditions ménagées et déshydraté à l'état gazeux par passage sur un catalyseur solide.

10. Procédé suivant l'une des revendications 7 a 9, caractérisé en ce que le catalyseur solide est choisi parmi les oxydes minéraux acides ou basiques, en particulier parmi $Al_2O_3$, $SiO_2$ et BaO.